# EUROPEAN PATENT SPECIFICATION

(11) **EP 3 723 943 B1**
(45) Date of publication and mention of the grant of the patent: **07.06.2023**
(21) Application number: 18887808.6
(22) Date of filing: 05.12.2018
(51) Int. Cl.: B25J 9/08, E05B 47/06, B25J 9/00, A61H 1/02

(54) **DRIVING ASSEMBLY FOR MOVING BODY PART**
ANTRIEBSANORDNUNG FÜR EIN BEWEGLICHES KÖRPERTEIL
ENSEMBLE D'ENTRAÎNEMENT POUR LE DÉPLACEMENT D'UNE PARTIE DE CORPS

(30) Priority: 11.12.2017 AU 2017904968; 13.01.2018 AU 2018900103
(43) Date of publication of application: 21.10.2020
(73) Proprietor: Ho, Sze Kit, Tsuen Wan, NT, HKSAR (CN)
(72) Inventor: Ho, Sze Kit, Tsuen Wan, NT, HKSAR (CN)
(74) Representative: Cabinet Chaillot
(86) International application number: PCT/IB2018/059676
(87) International publication number: WO 2019/116172

(56) References cited:
- CN-A- 104 688 392
- CN-A- 106 335 066
- CN-A- 106 828 674
- CN-U- 205 382 790
- CN-U- 206 633 029
- US-A- 5 376 091
- US-A1- 2009 069 942
- US-A1- 2010 305 717

## Description

### Technical Field

The present disclosure relates to a driving assembly, and more particularly to a driving assembly for moving or rotating, or assisting or resisting movement of, a first body part relative to a second body part, whereby enabling flexion, extension, pronation, supination, rotation, abduction, and adduction of the first body part relative to the second body part.

### Background Art

Existing technologies for manipulating body parts are usually configured for: positioning the motors/actuators directly on the joint rotation axial; or positioning a mechanical joint directly on the joint rotation axial and have the extension to be manipulated by actuators at a remote/distance site. Both designs require the body joint to be covered by motors and/or mechanical joints on one and/or both sides. Such an over-the-joint design requires the body part to be fitted in between the mechanical parts, and because of different limb circumferences, different sizes are needed. Sometimes the motors/actuators get into the way of the body movement. And most importantly, these prior arts are incapable to manipulate joints that have the axial physically blocked or covered.

Further, each year there are more than 18 millions people suffer from stroke and some of them lost some of the body functions or control over various body parts, such as limbs including fingers, hands, legs and the like, due to the damage of brain neurons. They need to practice the body functions in order to relearn the control thereof. In this regard, a manipulator capable of assisting control and movement for the impaired body parts, such as limbs, is highly desirable in the art.

Prior art documents include US 5 376 091 A that discloses a dynamic finger support with a driving assembly of the over-the-joint and invasive type and US 2010/0305717 A1 that discloses a wearable power assistive device for helping a user to move their hand, both including non-cascadable and non-driving assembly.

### Brief Summary of Invention

One aspect of the present invention relates to a driving assembly as defined in independent claim 1, for moving or rotating, or assisting or resisting movement of a first body part relative to a second body part. Optional features of this driving assembly are the subject-matter of dependent claims 2-5.

Another aspect of the present invention relates to a robotic hand or glove as defined in claims 8-10.

### Brief Description of Drawings

The present disclosure will be described in details below with reference to the accompanying drawings, in which:
Figures 1 and 1a are respectively the perspective and side view of an example driving assembly adapted for moving an associated component / object according to a preferred embodiment of the present disclosure;
Figures 2a-2d are respectively the partially cutaway view and perspective views of another example driving assembly adapted for moving an associated component / object according to a preferred embodiment of the present disclosure;
Figures 3a-3g are respectively the perspective views of other example driving assembly adapted for moving an associated component / object according to preferred embodiments of the present disclosure;
Figures 4a-4l are respectively the perspective views of other example driving assembly adapted for moving associated body parts according to preferred embodiments of the present disclosure;
Figures 5a-5c are respectively the perspective view and top view of another example driving assembly / glove adapted for moving associated body parts according to preferred embodiments of the present disclosure; and
Figure 6 is a perspective side view of a further example driving assembly for moving associated body parts according to a preferred embodiment of the present disclosure.

### Detailed Description of Invention

The present disclosure will now be described in further details with reference to the accompanying drawings and embodiments, so as to make the objects, technical solutions and advantages of the present disclosure more apparent.

According to the present disclosure, it provides a body joint manipulator devised to rotate body parts according to the joint axial, and allows the manipulation of body parts in which the body joints are inaccessible. The manipulator consists of an integration of an actuator, a transmission gear, a partial gear wheel as a moving arm and a housing preferably having an arc channel to guide the movement of the geared arm. The geared arm and/or arc channel have connectors attached to the body parts and/or connect to each other. The manipulator can be configured as a single joint manipulator or it could be cascaded for multiple joints manipulation or composite movement manipulation of a single joint.

In some embodiments, the technical solution of the present disclosure is able to manipulate a body joint without covering the joint axial, it is done by confining the movement within the arc channel where the rotation centre at the joint axial. Thus it is easy to mount and demount the manipulators on and from the body parts without requiring any fitting. For joints that are blocked by other body parts, the technical solution can manipulate the movement of the joints at ease. Another advantage of the technical solution is its cascadability. Multiple manipulators can be cascaded together through connectors of different lengths. Thus it is able to cover the whole body limb in one configuration.

According to the present disclosure, it provides a body joint manipulator which is capable to move or rotate body parts along the corresponding body joints without occupying the space at the joint axial. This manipulator constitutes of an actuator, a torque transmission gear, a partial gear wheel, a housing for the mechanical parts (actuator, the gear and partial gear wheel) combined with an arc channel or rail to guide the movement of the partial gear wheel (to be moved out or in the arc of the housing) and connectors at the tail of the housing and the head/tip of the geared wheel to allow fixture on the body parts as well as other mechanical parts (including cascade on another adjacent manipulator), where the actuator is a DC motor but can also be other actuators for example AC/DC motors, hydraulic, pneumatics, brush/brushless, or the like.

In some embodiments, the gear might be a worm gear but can also be other gears for example spur gear, bevel gear, belt gear, pulley gear, or the like. with one or combination of multiple gears or belts or cables; wherein the partial gear wheel is a worm wheel but can also be other gear wheel for example spur wheel, bevel wheel, belt wheel, pulley wheel, or the like; and wherein the partial gear wheel is a wheel with less than 360 degrees but more than 0 degree.

In other embodiments, the housing has an arc channel or rail with less than 360 degrees but more than 0 degree to allow rotation movement of the gear wheel with less than 360 degrees but more than 0 degree, wherein the movement of the partial gear wheel is confined by the arc channel of the housing and/or one or more rails on the side of the housing using but not limited to rods, bearings, rollers, or the like; and preferably the rails and/or the arc channel of the housing has one or more mechanical stoppers to confine the forward or backward movement of the gear wheel (move in or out of the housing).

In further embodiments, the housing has internal structure to confine the location of the motor, gear and gear wheel in order to maintain the center distances between the gear or gear wheel; wherein the housing can be one complete part or a combination of different parts joined together using but not limited to screws, nails, ultrasound, or the like; and/or wherein the gear, gear wheel and housing can be of different materials such as but not limited to plastics, metals, composite materials, or the like; and/or wherein the gear, gear wheel and housing can be manufactured by different methods such as but not limited to CNC, drill and milling, casting, injection molding, 3D printing, stamping, or the like.

In several example embodiments, wherein the connector can be located at any part of the housing and can be connected to any part of the manipulator of another copy and/or any part of the body; and/or wherein the connector can be of any length and of any shape to fit another manipulator and/or different body parts of different size.

According to the present disclosure, the manipulator can be applied on different body joints (but not limited to) such as shoulder, elbow, wrist, finger, hip, knee and ankle, or the like; and/or the manipulator can assist or resist movements (but not limited to) such as flexion, extension, abduction, adduction, rotation, grasp, open/close, or the like; and/or wherein the manipulator can be connected with another manipulator to provide additional rotation angle and/or more degree of freedom for one or more body joints; and/or wherein the manipulator can be cascaded together with one or more copies of this manipulator of different actuators, of different gears, of different sizes, of different range of motions, of different materials, of different shapes, of different connectors, of different fixtures, or the like; and/or wherein the manipulator can be connected to another manipulator with different arrangement (but not limited to) end to tip, tip to tip, end to end, or the like; and/or wherein the manipulator can be arranged with one or more manipulators serial or parallel, single or multiple according to different body parts or joints (but not limited to) such as a shoulder joint with flexion/extension and abduction/adduction and rotation, a wrist joint with flexion/extension and abduction/adduction, pronation/supination and rotation, a hand with multiple fingers joints with flexion/extension and abduction/adduction, a hip joint with flexion/extension and abduction/adduction and rotation, an ankle joint with flexion/extension and abduction/adduction and rotation, and other applicable body joints or the like.

According to the present disclosure, it further provides an exoskeleton robotic hand design having a body part manipulator or remote center manipulator (RCM), comprising a worm gear, a partial worm wheel, and preferably an arc rail channel to guide a remote center movement (RCM) for actuating fingers of a human hand. The robotic hand consists of more than 1 fingers with dual RCM for each finger to manipulate two finger joints - the PIP joint and MCP joint. The same manipulator can also actuate DIP and wrist joint if needed. This hand assists people with weak muscle strength and poor muscle coordination of hand functions to carry out normal hand functions with better strength and faster speed.

The RCM Hand is designed to actuate human hand fingers to assist the movement and enhance the strength. It consists of at least one manipulator for each finger. Each manipulator can actuate the finger movement according to its center of rotation. The RCM Hand can provide 10 degrees of freedom for 5 fingers manipulation. Each manipulator can provide greater than 90 degrees of freedom of rotation or range of motion. The RCM Hand is configured to provide assistive hand functions to help people to use their hand even though they have poor muscle strength and/or coordination.

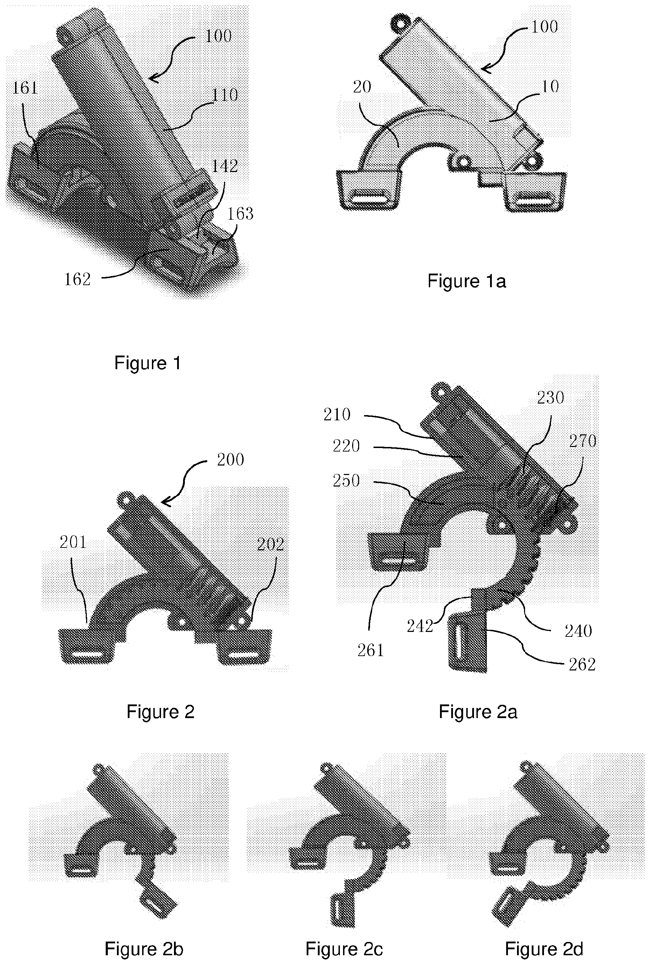

Referring to Figs. 1 and 1a, a perspective and a side view of an example driving assembly adapted for controlling an associated component / object according to a preferred embodiment of the present disclosure are illustrated, according to which the driving assembly comprises a manipulator unit 100 for moving a first part relative to a second part including a housing 110 for accommodating at least partly of an actuator, a torque / force transmission gear, a partial gear wheel operatively connected with each other and a first connector 161 arranged at a first / tail end of the manipulator unit for coupling with the first part and a second connector movably arranged at and coupled with a second / head end of the manipulator unit via its coupling mechanism 163 for coupling with the second part and preferably further coupled with and driven by a free end 142 of the partial gear wheel actuated by the actuator via the torque transmission gear for coupling and moving with the second part. The partial gear wheel will be completely received and concealed in the housing while in its idle state. It will be extending out or retracting into the housing via an opening arranged at the housing and preferably positioned at the second end of the manipulator unit while in its active state. As illustrated, the manipulator 100 might comprise a first portion 10 adapted for accommodating the actuator and the torque/force transmission gear and a second portion 20 having a part of a substantially semi-circular or curved cross-section for accommodating the partial gear wheel with a curved portion. In some embodiment, the first portion 10 is substantially in the form of a cylindrical casing and the second portion 20 is substantially in the form of a semi-circular or a curved casing for confining and guiding the motion of the partial gear wheel, wherein the cylindrical casing communicates with the curved casing for enabling a proper engagement between the partial gear wheel and the transmission gear.

Now referring to Figs. 2a-2d, partially cutaway view and perspective views of another example driving assembly adapted for moving an associated component / object according to a preferred embodiment of the present disclosure are illustrated. According to the present disclosure, the example driving assembly is adapted for moving or rotating, or assisting or resisting movement, or a first body part relative to a second body part (such as parts of a human / body joint) and preferably enabling flexion, extension, pronation, supination, rotation, abduction, and adduction of the first body part relative to the second body part, wherein the body joint includes but not limited to joints found in fingers, wrists, elbows, shoulders, knees, ankles, toes, neck, hips and human spine. The example driving assembly shown in Figs 2-2a comprises at least one manipulator unit 200 for body part including a housing 210 for accommodating at least partly of an actuator 220, a torque/force transmission gear 230, a partial gear wheel 240 operatively connected with each other and a first connector 261 arranged at a first/tail end 201 of the manipulator unit for coupling with the first body part and a second connector 262 movably arranged at a second/head end 202 of the manipulator unit for coupling with the second body part and preferably further coupled with and driven by a free end 242 of the partial gear wheel 240 actuated by the actuator via the torque transmission gear 230 for coupling and moving with the second body part, and preferably an arc channel or rail 250 for confining and/or guiding reciprocal or back and forth movement and preferably rotational movement of the partial gear wheel 240.

In several preferred embodiments, the actuator is selected from a group comprising a DC motor, an AC/DC motor, a hydraulic motor, a pneumatic motor, a brush/brushless motor, a piezo motor, a memory shaped alloy actuated by AC/DC electricity, batteries, chemicals, change of temperatures, change of pressures, lights or electromagnetism, sound or energy waves; and/or wherein the torque transmission gear is selected from a group comprising a worm gear, a spur gear, a bevel gear, a belt gear, a pulley gear, and a combination thereof; and/or wherein the partial gear wheel is selected from a group comprising a worm wheel, a spur wheel, a bevel wheel, a belt wheel, a pulley wheel, and a combination thereof; and/or wherein the partial gear wheel is a wheel with less than 360 degrees but more than 0 degree of rotation.

In some example embodiments, the free end 242 might be in the form of a profiled block with an inverted T-shape cross-section configured to be engageable with a counterpart of the coupling mechanism (such as a groove of a matched cross-section, resembled to and/or denoted by numeral163 in Fig. 1) of the second connector via tight fit and they could preferably further engaged with each other via a conventional screwing mechanism or coupling mechanism (not shown) well known in the art.

In several embodiments, the manipulator unit further comprises one or more stoppers arranged at, and preferably adjacent to a tail end of, the partial gear wheel, the arc channel or rail, and/or the housing, for confining movement and/or providing safety limits for movement of the partial gear wheel operated in both idle and active states.

In some of further embodiments, the manipulator unit further comprises a sensor 270 configured to measure and feedback a rotation angle or load force of the torque/force transmission gear 230 and/or the partial gear wheel 240 to enable an automatic adjustment of motion of associated body parts controlled by the driving assembly.

In other embodiments, the manipulator unit further comprises one or more sensors and/or one or more controllers arranged at or in the housing to measure or control / process data related to positions, angles of rotation, degrees of movement, or orientations of the free end and/or the second connector, forces or pressures exerted or received by the free end and/or the second connector, ambient sound, light, and/or temperature. In the embodiments, measured and output or feedback data generated by the sensors and/or the controllers are communicated or transmitted, preferably to a remote / external terminal, via one or more of wired and/or wireless technologies and protocols including Bluetooth, Wifi, Infrared, Zigbee, GSM, LTE, and a combination thereof.

According to the present disclosure, the second connector 262 is configured to be operated interchangeably between an idle/inactive state in which the second connector is positioned at or adjacent to the second end 202 and an active state in which the second connector is driven to move away from or move toward the second end 202 preferably along a circumferential direction by the partial gear wheel 240 or to rotate a predetermined or desired first angle about a first rotation axis defined by a curvature of the partial gear wheel and preferably the arc channel or rail in response to a preset instruction or a received real-time instruction, so as to drive the second body part coupled therewith to rotate / pivot a predetermined or desired second angle about a body joint / second rotation axis (not shown) positioned between the first and the second body parts. Preferably, the first rotation axis is substantially parallel to the second rotation axis. In some embodiments, the first rotation axis is substantially coinciding with the second rotation axis for enhancing or optimizing the driving efficiency thereof.

As can be seen from Figs. 2b-2d, the free end 242 of the partial gear wheel 240 or the second connector 262 is driven to move away from the second end 202 along the circumferential direction confined by the partial gear wheel 240 and/or preferably the arc channel or rail 250 to rotate respectively 45, 90, and 135 degrees of angle about the rotation axis defined by a curvature of the partial gear wheel and/or preferably the arc channel or rail in response to a preset instruction or a received real-time instruction provided by the driving assembly, wherein nearly one fourth, a half, and the whole of the partial gear wheel 240 has been driven and extended out from the housing 210 or the arc channel 250, such that the associated body parts will be driven accordingly to perform a desired motion.

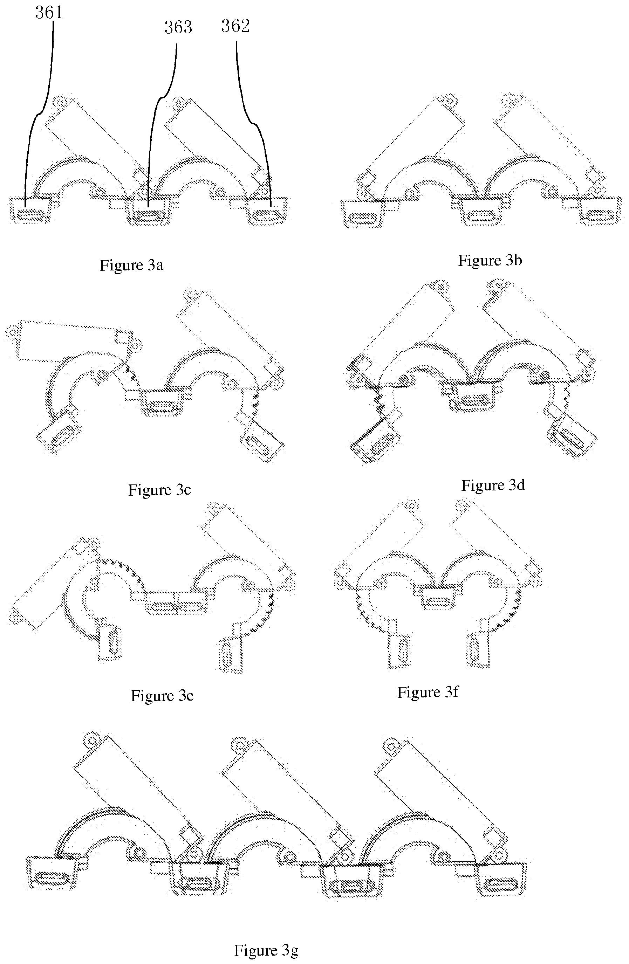

Now referring to Figs. 3a-3g, the perspective views of other example driving assembly according to preferred embodiments of the present disclosure are illustrated. According to the present disclosure, a plurality of manipulator units of identical dimension or of different dimensions might be arranged in serial and/or parallel connection with each other; wherein respective first connectors of the plurality of manipulator units are configured to couple with each other and/or with same first body part and respective second connectors of the plurality of manipulator units are configured to couple with each other and/or with same second body part for providing extra degrees of freedom of rotation or motion for the second body part in relation to the first body part.

In some embodiments, the second connector of one of the plurality of manipulator units is configured to couple with the first connector of other one of the plurality of manipulator units, so as to provide additional rotation angle and/or extra degrees of freedom of motion for one or more body parts or joints coupled with and driven singly/respectively or collectively by one or more of the plurality of manipulator units.

In yet still other embodiments, an adjacent pair of a first and a second manipulator units are coupled with each other via an interconnection of the second connector of the first manipulator unit and the first connector of the second manipulator unit, an interconnection of the first connector of the first manipulator unit and the second connector of the second manipulator unit, an interconnection of the first connector of the first manipulator unit and the first connector of the second manipulator unit, or an interconnection of the second connector of the first manipulator unit and the second connector of the second manipulator unit.

Fig. 3a has illustrated a head to tail or first configuration having two cascaded manipulators formed by connecting the head end of one of the manipulator units to the tail end of another manipulator unit via a first connector or a second connector acting as an intermediate connector 363, such that two manipulators will equip with only three connectors 361, 362, 363 in total; and Fig. 3b has illustrated a tail to tail or second configuration having two cascaded manipulators formed by connecting together the tail ends thereof; wherein the respective free ends of the partial gear wheels are both in an idle / inactive position and are driven to rotate along a circumferential direction confined by the partial gear wheel by 0 degree of angle.

Fig. 3c has illustrated the first configuration shown in Fig. 3a; and Fig. 3d has illustrated the second configuration shown in Fig. 3b; wherein each of free ends of the partial gear wheels is in an active position and is driven to move along a circumferential direction confined by the partial gear wheel and rotate by 45 degrees of angle, and thus the connector 362 positioned at the right hand side is rotated by 90 degrees of angle in total with respect to the connector 361 positioned at the left hand side.

Fig. 3e has illustrated the first configuration shown in Fig. 3a; and Fig. 3f has illustrated the second configuration shown in Fig. 3b; wherein each of free ends of the partial gear wheels is in an active position and is driven to move along a circumferential direction confined by the partial gear wheel and rotate by 90 degrees of angle, and thus the connector 362 positioned at the right hand side is rotated by 180 degrees of angle in total with respect to the connector 361 positioned at the left hand side. Further, the intermediate connector in Fig. 3e is formed by interconnecting two connectors, namely the second connector of the left manipulator unit and the first connector of the right manipulator unit; wherein these two connectors can be also connected via an intermediate connection member such as a connection rod (not shown) to lengthen the distance between the left manipulator unit and the right manipulator unit.

In some embodiments, three or more manipulators could be cascaded in series or parallel connection with each other, such as another head to tail configuration (or third configuration) as shown in Fig. 3g, which might be adapted to a specific application / joint with many articulation surfaces.

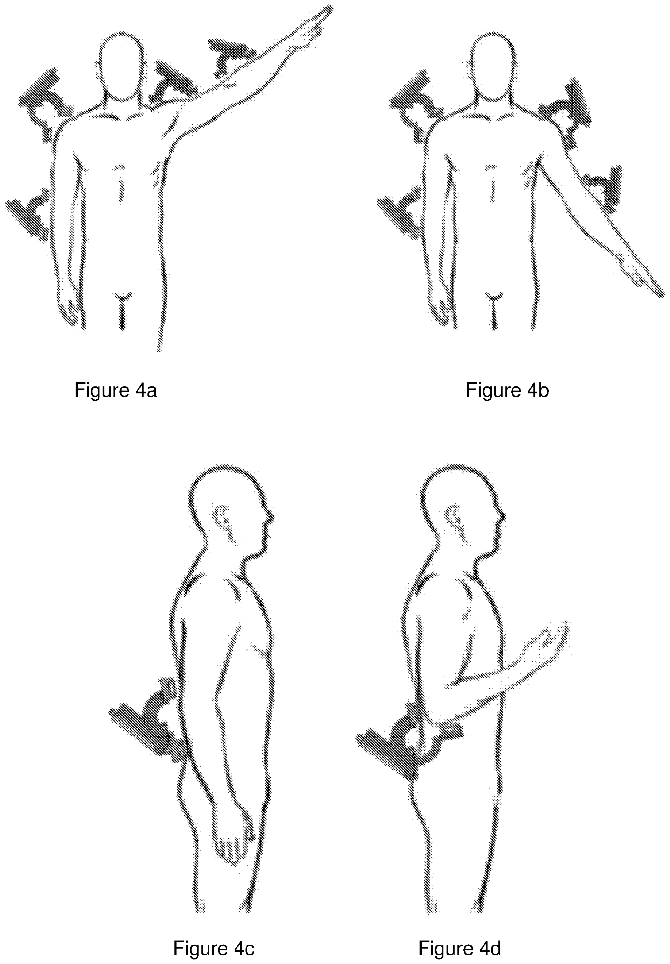

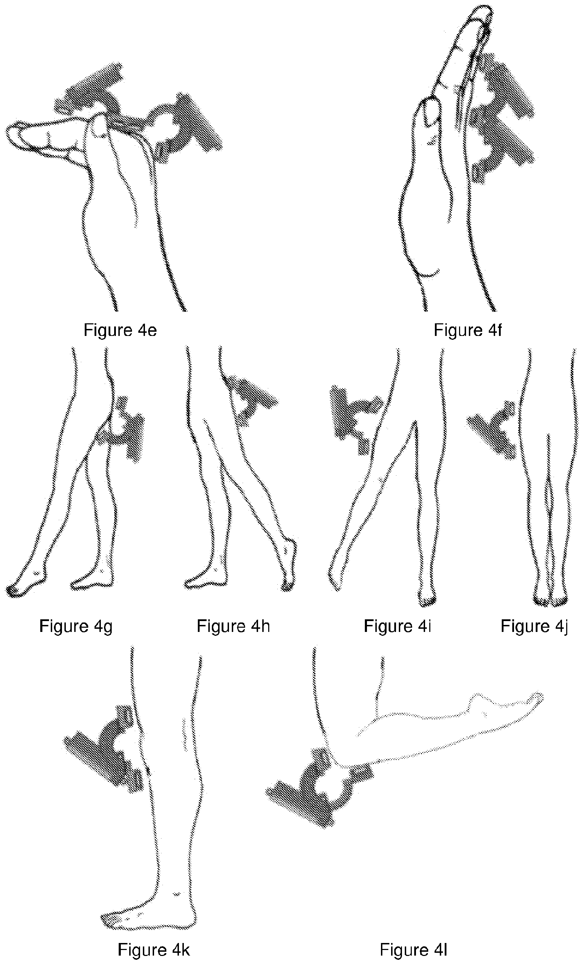

Figures 4a-4l are respectively the perspective views of other example driving assembly adapted for moving associated body parts according to preferred embodiments of the present disclosure. As shown in Figs 4a-4b, four manipulator units are configured to be positioned in an upper limb covering elbow and shoulder of both arms. In this embodiment, the movement or abduction and adduction of the left arm are controlled by the manipulator unit positioned on the left shoulder.

Referring to Figs 4c-4d, one manipulator unit is positioned at the back of the right arm elbow, wherein the first connector arranged at the first end of the manipulator unit is connected with the upper arm and the movable second connector arranged at the second end of the manipulator unit is connected with the forearm, such that various movement, namely the flexion and extension, of the right elbow are driven and controlled by the respective operations of the manipulator unit.

Now referring to Figs 4e-4f, two manipulator units in series connection or head to tail configuration are positioned at the back of the hand and a finger, wherein the first connector arranged at the first end of the right manipulator unit is connected with the hand and the movable second connector arranged at the second end of the left manipulator unit is connected with the finger, such that various movement, such as the flexion and extension, of the PIP and MCP joints of the finger are controllable by the respective operations of the left and/or the right manipulator units.

In some embodiments as shown in Figs 4g-4j, one or more manipulator units might be positioned at the back or lateral side of the hip joint, wherein the first connector arranged at the first end of the manipulator unit might be connected with the buttock and the movable second connector arranged at the second end of the manipulator unit might be connected with the upper leg, such that various movement, namely the abduction/adduction, flexion/extension, of the hip joint are driven and controlled by the respective operations of the one or more manipulator units.

In some embodiments as illustrated in Figs 4k-4l, a manipulator unit is positioned at the knee joint, wherein the first connector arranged at the first end of the manipulator unit is connected with the upper leg and the movable second connector arranged at the second end of the manipulator unit is connected with the lower leg, such that various movement, namely the flexion and extension, of the knee joint are driven and controlled by the respective operations of the manipulator unit.

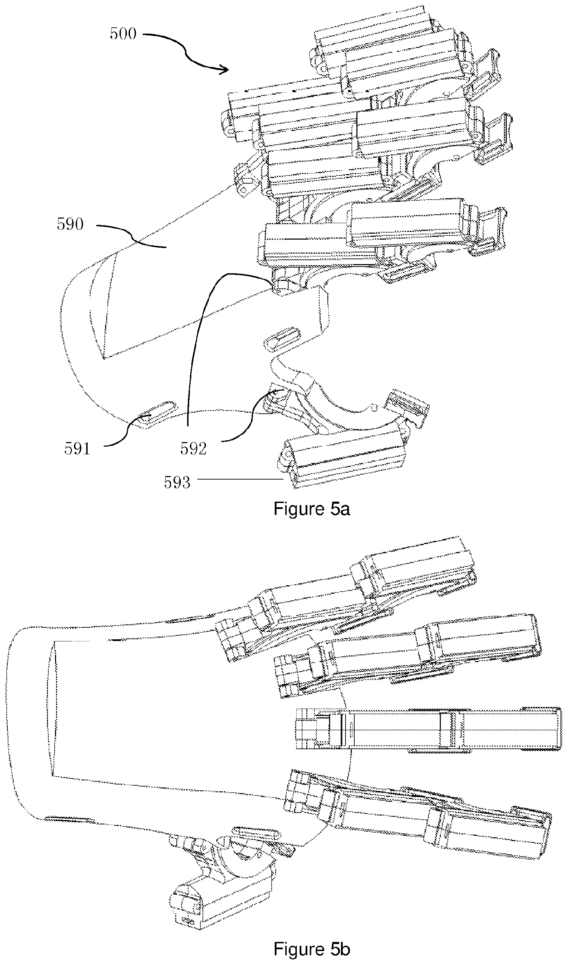

Now referring to Figs. 5a-5b, the perspective view and top view of another example driving assembly in the form of a robotic hand or glove adapted for moving associated hand and fingers according to preferred embodiments of the present disclosure are shown. The robotic hand or glove 500 is adapted for moving or rotating, or assisting or resisting movement of, a human hand and/or its fingers comprising a foregoing driving assembly, and further comprising a palm plate for mounting the robotic hand or glove on the human hand wrist, and/or a forearm and for mounting a plurality of manipulator units 593 for use with and manipulation of a plurality of fingers of the human hand, wherein each of the plurality fingers is coupled with and actuated by one or more cascaded manipulator units configured for and capable of providing one or more degrees of freedom of motion.

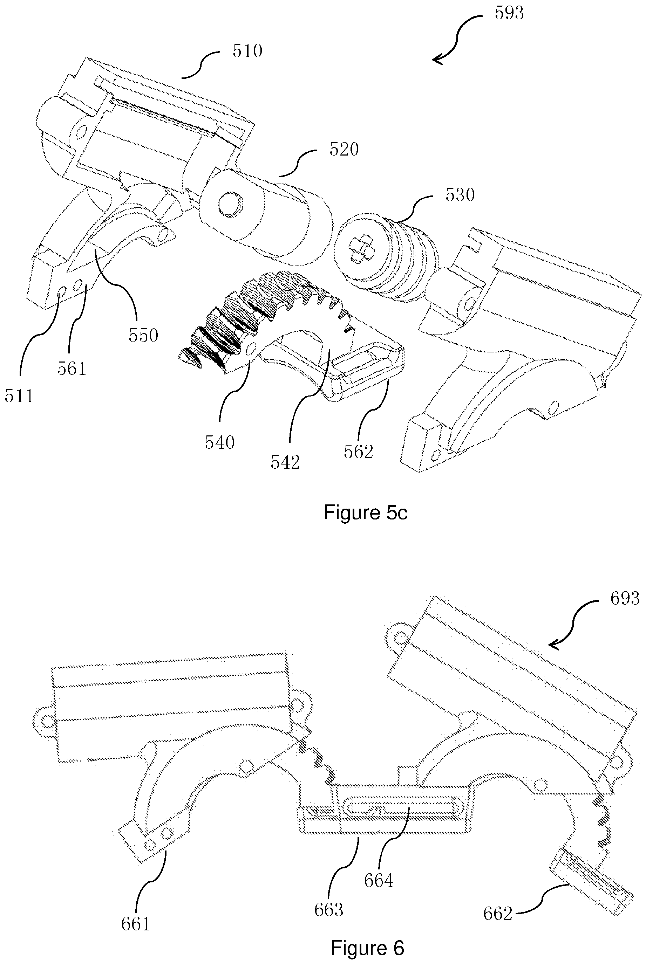

Referring to Fig. 5c, the manipulator unit 593 is similar to the one depicted in Fig. 2a and comprises a housing 510 for accommodating at least partly of an actuator 520, a torque/force transmission gear 530, a partial gear wheel 540 operatively connected with each other and a first connector 561 arranged at a first/tail end of the manipulator unit for coupling with the first body part and/or the palm plate 590 and a second connector 562 movably arranged at a second/head end of the manipulator unit for coupling with the second body part and/or an adjacent manipulator unit and preferably further coupled with and driven by a free end 542 of the partial gear wheel 540 actuated by the actuator via the torque transmission gear 530 for coupling and moving with the second body part, and preferably an arc channel or rail 550 for confining and/or facilitating the guiding of reciprocal or back and forth movement and preferably rotational movement of the partial gear wheel 540.

The housing might be engaged with the palm plate via its coupling mechanism 511, which might be a conventional screwing or fastening mechanism.

In several preferred embodiments, the robotic hand or glove further comprises one or more sensors or sensing devices including an electromyography sensor for measuring electrical activity of muscles of the human hand and fingers, a force sensitive resistor for measuring or gathering information about pressure at each fingertip of the human hand, an accelerometer for measuring acceleration forces of the human hand and fingers, and/or a gyroscope for measuring or maintaining orientation and angular velocity of the human hand and fingers.

In other embodiments, the robotic hand or glove further comprises a micro-controller configured to control / manipulate, coordinate, and/or synchronize movement of the plurality of manipulator units in response to data measured and provided by the one or more sensors for effecting desired operations of the robotic hand for assisting or resisting movement of the human hand.

In yet still other embodiments, the palm plate further comprises at least one first attachment connector 591 having a first engagement member arranged at or in proximity of a proximal end of the palm plate for coupling with a human wrist and/or forearm and/or at least one second attachment connector 592 having a second engagement member arranged at or in proximity of a distal end of the palm plate for coupling with the first connector of a proximal one of the plurality of manipulator units.

In further example embodiments, two manipulator units arranged in a cascaded manner are coupling with one of the fingers or thumb of the human hand to provide 2 degrees of freedom and actuate rotation of one or more finger joints including PIP joint and/or MCP joint to provide flexion and/or extension of the finger or thumb; and/or two or three manipulator units arranged in a cascaded manner are coupling with other one of the fingers, including index finger, middle finger, ring finger, and little finger, of the human hand to provide 2 or 3 degrees of freedom and actuate rotation of one or more finger joints including PIP joint, MCP joint, and/or DIP joint; or thumb of the human hand to provide 2 or 3 degrees of freedom and actuate rotation of one or more finger joints including IP joint, MCP joint, and/or CMC joint; and/or wherein extra one or more manipulators arranged in standalone or combination with others are coupling with the thumb and other fingers to provide extra degree of freedom for abduction and/or adduction.

In several embodiments, each of manipulator units of the robotic hand or glove is configured to provide itself or a segment of a finger of the human hand with rotation movement preferably greater than 0 degree and more preferably less than 180 degree.

According to the present disclosure, the robotic hand or glove 500 can provide more than 2 degree of freedoms for each finger. It employs one or more manipulator / manipulator units to actuate one or more fingers, wherein each finger has more than 1 manipulator to actuate the rotation of the finger joint such as PIP joint and/or MCP joint. Each manipulator is configured to manipulate the finger and to conform the rotation remotely at finger joints, wherein the degrees of rotation (range of motion) and/or the finger joint torque generate by a human being are measured with sensors embedded therein. The arc channel or rail is configured to facilitate the guiding of the movement of the gear wheel and the manipulator further comprises a limited stopper to provide safety limits for the motion of the gear wheel. Two or more manipulators can be connected with each other in different configurations or arrangements such as head to head, tail to tail, head to tail or tail to head as previously illustrated. Each manipulator might equip with a connector at the end of gear wheel and the tail of the housing in order to attach to fingers to be manipulated. The palm plate is configured to secure the manipulators of different fingers and it could be securely attached to the back of a human hand.

As described above, the robotic hand or glove 500 may have attachment connectors (such as 591 depicted in Fig. 5a) for attaching to different fingers and wrist in order to manipulator these body parts. The connector of a manipulator can be attached to a human body or connected to another manipulator. The robotic hand or glove might consist of finger pad for securing the finger on the manipulator, and micro-controller to manipulate the movement of these manipulators; and other sensors such as Electromyography signals, accelerometer and gyroscope, FSR, or the like; and it might be connected wirelessly to other terminals for control and data transfer. In some embodiments, each manipulator can be configured to provide rotation movement for greater than 90 degree but less than 180 degree.

Now referring to Fig. 6, a perspective side view of a further example driving assembly for moving associated body parts according to a preferred embodiment of the present disclosure is illustrated, according to which two manipulators 693 are cascaded for assisting movement of or control over a body part, such as a finger, wherein the tail end of the leftmost connector 661 of the left manipulator is configured to be mountable at a first body part or a palm plate, while its head end and the tail end of the right manipulator are connected or coupled in a manner resembling to the same illustrated in Figs. 3a via an intermediate connector 663 with a prolonged length / dimension. The intermediate connector 663 comprises a first and a second coupling members spaced apart in a length direction and arranged at opposing ends for coupling with respective ends of the left and right manipulators or a free end of a partial gear wheel thereof; and an opening 664, preferably a through slot, extended along a width direction adapted for insertion of a fastening member such as a band / rope for winding on a second body part (or a finger) for positioning / mounting the driving assembly properly over a finger or a corresponding finger joint (not shown). The dimension or the length and/or the width of the intermediate connector might be adapted to a predetermined dimension of the body part to provide or form an optimized joint with a desirable stiffness between the left and right manipulators for enabling / facilitating optimized operations thereof; and it might also differs from the same of the leftmost connector 661 arranged at the tail end of the left manipulator and/or the rightmost connector 662 arranged at the head end of the right manipulator, as illustrated in the figure. In some embodiments, the left and right manipulators are integrated with the intermediate and/or the rightmost connectors.

The present disclosure is described according to specific embodiments, but those skilled in the art will appreciate that various changes and equivalents might be made without departing from the scope of the present disclosure. In addition, many modifications might be made to the present disclosure without departing from the scope of the invention in order to adapt to specific circumstances or components of the present disclosure. Accordingly, the present disclosure is not limited to the specific embodiments disclosed herein, and shall include all embodiments falling within the scope of the claims.

## Claims

1. A driving assembly for moving or rotating, or assisting or resisting movement of, a first body part relative to a second body part and preferably enabling flexion, extension, pronation, supination, rotation, abduction, and adduction of the first body part relative to the second body part, comprising at least one remote center and cascadable manipulator unit (100, 200, 593) for body part including a housing (110, 210, 510) for accommodating at least partly of an actuator (220, 520), a torque/force transmission gear (230, 530), a partial gear wheel (240, 540) operatively connected with each other, a first connector (161, 261, 361, 561) arranged for coupling with the first body part, a second connector (162, 262, 362, 562) coupled with the partial gear wheel (240, 540) and arranged for coupling with the second body part and further configured to be driven by the partial gear wheel actuated by the actuator (220, 520) via the torque transmission gear (230, 530) for coupling and moving with the second body part, and an arc channel or rail (250, 550) for confining and/or guiding reciprocal or back and forth remote center rotational movement of the partial gear wheel (240, 540),
wherein the second connector (162, 262, 362, 562) is configured to be driven by the partial gear wheel (240, 540) to rotate a predetermined or desired first angle about a rotation axis defined by a curvature of the partial gear wheel and the arc channel or rail (250, 550) in response to a preset instruction or a received real-time instruction, so as to drive the second body part coupled therewith to rotate a predetermined or desired second angle about a body joint positioned between the first and the second body parts,
wherein the cascadable manipulator unit is capable of rotating the second body part without occupying axial space of the body joint;
and wherein the cascadable manipulator unit is cascadable with its duplicate or another cascadable manipulator unit of different actuators, of different gears, of different sizes, of different ranges of motion, or of different materials, for multiple body joints manipulation or composite movement manipulation of a single body joint.

2. The driving assembly according to claim 1, comprising a plurality of cascadable manipulator units (100, 200, 593) of identical dimension or of different dimensions arranged in serial and/or parallel connection with each other; wherein respective first connectors (161, 261, 361, 561) of the plurality of manipulator units (100, 200, 593) are configured to couple with each other and/or with same first body part and respective second connectors (162, 262, 362, 562) of the plurality of manipulator units are configured to couple with each other and/or with same second body part for providing extra degrees of freedom of rotation or motion for the second body part in relation to the first body part; and/or
wherein the second connector (162, 262, 362, 562) of one of the plurality of manipulator units (100, 200, 593) is configured to couple with the first connector (161, 261, 361, 561) of other one of the plurality of manipulator units, so as to provide additional rotation angle and/or extra degrees of freedom of motion for one or more body parts or joints coupled with and driven singly/respectively or collectively by one or more of the plurality of manipulator units.

3. The driving assembly according to claim 1 or 2, further comprising one or more stoppers arranged at, and preferably adjacent to a tail end of, the partial gear wheel, the arc channel or rail, and/or the housing for confining movement and/or providing safety limits for movement of the partial gear wheel.

4. The driving assembly according to claim 1 or 2, wherein the actuator is selected from a group comprising a DC motor, an AC/DC motor, a hydraulic motor, a pneumatic motor, a brush/brushless motor, a piezo motor, a memory shaped alloy actuated by AC/DC electricity, batteries, chemicals, change of temperatures, change of pressures, lights or electromagnetism, sound or energy waves; and/or
wherein the torque transmission gear is selected from a group comprising a worm gear, a spur gear, a bevel gear, a belt gear, a pulley gear, and a combination thereof; and/or
wherein the partial gear wheel is selected from a group comprising a worm wheel, a spur wheel, a bevel wheel, a belt wheel, a pulley wheel, and a combination thereof; and/or
wherein the partial gear wheel is a wheel with less than 360 degrees, preferably less than 135 degrees, but more than 0 degree of rotation.

5. The driving assembly according to claim 1, wherein the manipulator unit (100, 200, 593) further comprises one or more sensors and/or one or more controllers arranged at or in the housing to measure or control / process data related to positions, angles of rotation, degrees of movement, or orientations of the second connector (162, 262, 362, 562), forces or pressures exerted or received by the second connector (162, 262, 362, 562), ambient sound, light, and/or temperature;
wherein measured and output or feedback data generated by the sensors and/or the controllers are communicated or transmitted, preferably to a remote / external terminal, via one or more of wired and/or wireless technologies and protocols including Bluetooth, Wifi, Infrared, Zigbee, GSM, LTE, and a combination thereof.

6. The driving assembly according to claim 1, wherein an adjacent pair of a first and a second cascadable manipulator units (100, 200, 593) are coupled with each other via an interconnection of the second connector (162, 262, 362, 562) of the first manipulator unit and the first connector (161, 261, 361, 561) of the second manipulator unit, an interconnection of the first connector (161, 261, 361, 561) of the first manipulator unit and the second connector (162, 262, 362, 562) of the second manipulator unit, an interconnection of the first connector (161, 261, 361, 561) of the first manipulator unit and the first connector (161, 261, 361, 561) of the second manipulator unit, or an interconnection of the second connector (162, 262, 362, 562) of the first manipulator unit and the second connector (162, 262, 362, 562) of the second manipulator unit.

7. A robotic hand or glove (500) for moving or rotating, or assisting or resisting movement of, a human hand and/or its fingers comprising a driving assembly according to any one of claims 1-6, further comprising a palm plate for mounting the robotic hand or glove on the human hand wrist, and/or a forearm and for mounting at least one remote center and cascadable manipulator units (100, 200, 593) for use with and manipulation of at least one finger of the human hand, wherein each of the plurality fingers is coupled with and actuated by one or more manipulator units (100, 200, 593) configured for and capable of providing one or more degrees of freedom; and
one or more sensors or sensing devices including an electromyography sensor for measuring electrical activity of muscles of the human hand and fingers, a force sensitive resistor for measuring or gathering information about pressure at each fingertip of the human hand, an accelerometer for measuring acceleration forces of the human hand and fingers, and/or a gyroscope for measuring or maintaining orientation and angular velocity of the human hand and fingers;
a micro-controller configured to control / manipulate, coordinate, and/or synchronize movement of the plurality of manipulator units (100, 200, 593) in response to data measured and provided by the one or more sensors for effecting desired operations of the robotic hand for assisting or resisting movement of the human hand.

8. The robotic hand or glove according to claim 7, wherein the palm plate further comprises at least one first attachment connector having a first engagement member arranged at or in proximity of a proximal end of the palm plate for coupling with a human wrist and/or forearm and/or at least one second attachment connector having a second engagement member arranged at or in proximity of a distal end of the palm plate for coupling with the first connector (161, 261, 361, 561) of a proximal one of the plurality of manipulator units (100, 200, 593).

9. The robotic hand or glove according to claim 7, wherein two manipulator units (100, 200, 593) arranged in a cascaded manner are coupling with one of the fingers or thumb of the human hand to provide 2 degrees of freedom and actuate rotation of one or more finger joints including PIP joint and/or MCP joint to provide flexion and/or extension of the finger or thumb; and/or two or three manipulator units (100, 200, 593) arranged in a cascaded manner are coupling with other one of the fingers, including index finger, middle finger, ring finger, and little finger, of the human hand to provide 2 or 3 degrees of freedom and actuate rotation of one or more finger joints including PIP joint, MCP joint, and/or DIP joint; or thumb of the human hand to provide 2 or 3 degrees of freedom and actuate rotation of one or more finger joints including IP joint, MCP joint, and/or CMC joint; and/or wherein extra one or more manipulators arranged in standalone or combination with others are coupling with the thumb and other fingers to provide extra degree of freedom for abduction and/or adduction.

10. The robotic hand or glove according to any one of claims 7-9, wherein each of manipulator units (100, 200, 593) is configured to provide itself or a segment of an associated finger of the human hand with rotation movement preferably greater than 0 degree and more preferably less than 180 degree.

## Patentansprüche

1. - Antriebsanordnung zum Bewegen oder Drehen oder Unterstützen oder Verhindern der Bewegung eines ersten Körperteils mit Bezug auf ein zweites Körerteil und vorzugsweise Ermöglichen der Beugung, Extension, Pronation, Supination, Rotation, Abduktion und Adduktion des ersten Körperteils mit Bezug auf das zweite Körerteil, umfassend mindestens ein entferntes Zentrum und abstufbare Manipulationseinheit (100, 200, 593) für das Körperteil, darin eingeschlossen ein Gehäuse (110, 210, 510) zur mindestens teilweisen Aufnahme eines Aktuators (220, 520), eines Drehmoment-/Kraftübertragungsgetriebes (230, 530), eines teilweisen Getrieberads (240, 540), die in Betrieb miteinander verbunden sind, eines ersten Verbinders (161, 261, 361, 561), der angeordnet ist, um mit dem ersten Körperteil zu koppeln, einen zweiten Verbinder (162, 262, 362, 562), der mit dem teilweisen Getrieberad (240, 540) gekoppelt und angeordnet ist, um mit dem zweiten Körperteil zu koppeln, und weiter konfiguriert, um vom teilweisen Getrieberad angetrieben zu werden, das vom Aktuator (220, 520) mit Hilfe des Drehmoment-/Kraftübertragungsgetriebes (230, 530) angetrieben wird, um mit dem zweiten Körperteil zu koppeln und sich zu bewegen, und einen bogenförmigen Kanal oder eine bogenförmigen Schiene (250, 550), um die eine Drehbewegung des entfernten Zentrums des teilweisen Getrieberads (240, 540) einzuschränken und/oder reziprok oder hin- und zurück zu führen,
wobei der zweite Verbinder (162, 262, 362, 562) konfiguriert ist, um vom teilweisen Getrieberad (240, 540) angetrieben zu werden, um mit einem vorbestimmten oder gewünschten ersten Winkel um eine Drehachse zu drehen, definiert von einer Krümmung des teilweisen Getrieberads und des bogenförmigen Kanals oder der bogenförmigen Schiene (250, 550) in Reaktion auf eine voreingestellte Anweisung oder erhaltene Echtzeit-Anweisung, um das zweite Körperteil, das damit gekoppelt ist, anzutreiben, um mit einem vorbestimmten oder gewünschten zweiten Winkel um ein Körpergelenk zu drehen, das zwischen dem ersten und dem zweiten Körperteil positioniert ist,
wobei die abstufbare Manipulationseinheit dazu in der Lage ist, das zweite Körperteil zu drehen, ohne den axialen Raum des Körpergelenks einzunehmen;
und wobei die abstufbare Manipulationseinheit mit ihrem Duplikat oder einer anderen abstufbaren Manipulationseinheit von verschiedenen Aktuatoren, mit verschiedenen Getrieben, mit verschiedenen Größen, mit verschiedenen Bewegungsbereichen, oder mit verschiedenen Materialien für zahlreiche Körpergelenkmanipulationen oder zusammengesetzte Bewegungsmanipulationen eines einzigen Körpergelenks abstufbar ist.

2. - Antriebsanordnung nach Anspruch 1, umfassend eine Vielzahl von abstufbaren Manipulationseinheiten (100, 200, 593) mit identischer Abmessung oder verschiedenen Abmessungen, die in serieller und/oder paralleler Verbindung miteinander angeordnet sind; wobei jeweilige erste Verbinder (161, 261, 361, 561) der Vielzahl von Manipulationseinheiten (100, 200, 593) konfiguriert sind, um miteinander und/oder mit dem gleichen ersten Körperteil zu koppeln und entsprechende zweite Verbinder (162, 262, 362, 562) der Vielzahl von Manipulationseinheiten konfiguriert sind, um miteinander und/oder mit dem gleichen zweiten Körperteil zu koppeln, um zusätzliche Freiheitsgrade der Drehung oder Bewegung für das zweite Körperteil mit Bezug auf das erste Körperteil bereitzustellen; und/oder
wobei der zweite Verbinder (162, 262, 362, 562) einer der Vielzahl von Manipulationseinheiten (100, 200, 593) konfiguriert ist, um mit dem ersten Verbinder (161, 261, 361, 561) einer anderen der Vielzahl von Manipulationseinheiten zu koppeln, um einen zusätzlichen Drehungswinkel und/oder zusätzliche Freiheitsgrade der Bewegung für ein oder mehrere Körperteile oder Gelenke, gekoppelt mit und angetrieben allein/bzw. oder zusammen durch eine oder mehrere der Vielzahl von Manipulationseinheiten bereitzustellen.

3. - Antriebsanordnung nach Anspruch 1 oder 2, weiter umfassend einen oder mehrere Stopper, die an und vorzugsweise benachbart einem hinteren Ende des teilweisen Getrieberads, des bogenförmigen Kanals oder der bogenförmigen Schiene und/oder des Gehäuses angeordnet sind, um die Bewegung einzuschränken und/oder Sicherheitsgrenzen für die Bewegung des teilweisen Getrieberads bereitzustellen.

4. - Antriebsanordnung nach Anspruch 1 oder 2, wobei der Aktuator ausgewählt ist aus einer Gruppe, umfassend einen DC-Motor, einen AC/DC-Motor, einen hydraulischen Motor, einem pneumatischen Motor, einen Bürsten-/bürstenlosen Motor, einen Piezomotor, eine Formgedächtnislegierung, betätigt durch AC/DC-Elektrizität, Batterien, Chemikalien, Temperaturänderungen, Änderungen von Druck, Licht oder Elektromagnetismus, Schall- oder Energiewellen; und/oder
wobei das Drehmomentübertragungsgetriebe ausgewählt ist aus einer Gruppe, umfassend ein Schneckengetriebe, ein Stirnradgetriebe, ein Kegelradgetriebe, ein Riemengetriebe, ein Riemenscheibengetriebe und eine Kombination davon; und/oder wobei das teilweise Getrieberad ausgewählt ist aus einer Gruppe, umfassend ein Schneckenrad, ein Stirnrad, ein Kegelrad, ein Riemenrad, ein Riemenscheibenrad und eine Kombination davon; und/oder
wobei das teilweise Getrieberad ein Rad mit weniger als 360 Grad, vorzugsweise weniger als 135 Grad aber mehr als 0 Grad Rotation ist.

5. - Antriebsanordnung nach Anspruch 1, wobei die Manipulationseinheit (100, 200, 593) weiter einen oder mehrere Sensoren und/oder eine oder mehrere Steuerungen umfasst, die am oder im Gehäuse angeordnet sind, um Daten zu messen oder zu steuern/zu verarbeiten, die sich auf Positionen, Rotationswinkel, Bewegungsgrade, oder Ausrichtungen des zweiten Verbinders (162, 262, 362, 562), Kräfte oder Drücke, die vom zweiten Verbinder (162, 262, 362, 562) ausgeübt oder erhalten werden, Umgebungsgeräusche, Licht und/oder Temperatur bezieht, wobei gemessene und ausgegebene oder Feedback-Daten, erzeugt von den Sensoren und/oder den Steuerungen kommuniziert oder übertragen werden, vorzugsweise an ein entferntes/externes Endgerät, über ein oder mehrere verdrahtete und/oder drahtlose Technologien und Protokolle, darin eingeschlossen Bluetooth, Wifi, Infrared, Zigbee, GSM, LTE und eine Kombination davon.

6. - Antriebsanordnung nach Anspruch 1, wobei ein benachbartes Paar von ersten und zweiten abstufbaren Manipulationseinheiten (100, 200, 593) miteinander über eine Verbindung des zweiten Verbinders (162, 262, 362, 562) der ersten Manipulationseinheit und des ersten Verbinders (161, 261, 361, 561) der zweiten Manipulationseinheit einer Verbindung des ersten Verbinders (161, 261, 361, 561) der ersten Manipulationseinheit und des zweiten Verbinders (162, 262, 362, 562) der zweiten Manipulationseinheit, einer Verbindung des ersten Verbinders (161, 261, 361, 561) der ersten Manipulationseinheit und des ersten Verbinders (161, 261, 361, 561) der zweiten Manipulationseinheit oder einer Verbindung des zweiten Verbinders (162, 262, 362, 562) der ersten Manipulationseinheit und des zweiten Verbinders (162, 262, 362, 562) der zweiten Manipulationseinheit gekoppelt sind.

7. - Roboterhand oder Roboterhandschuh (500) zum Bewegen oder Drehen oder Unterstützen oder Verhindern der Bewegung einer menschlichen Hand und/oder ihrer Finger, umfassend eine Antriebseinheit nach einem der Ansprüche 1 - 6, weiter umfassend eine Handflächenplatte zur Montage der Roboterhand oder des Roboterhandschuhs auf dem menschlichen Handgelenk und/oder Unterarm und zur Montage mindestens eines entfernten Zentrums und abstufbarer Manipulationseinheiten (100, 200, 593) zur Verwendung mit und Manipulation von mindestens eines Fingers der menschlichen Hand, wobei jedes der Vielzahl von Fingern mit einer oder mehreren Manipulationseinheiten (100, 200, 593) gekoppelt und davon betätigt wird, die konfiguriert und dazu in der Lage ist, einen oder mehrere Freiheitsgrade bereitzustellen; und
einen oder mehrere Sensoren oder Sensorvorrichtungen, darin eingeschossen einen Elektromyographiesensor, um die elektrische Aktivität von Muskeln der menschlichen Hand und Finger zu messen, einen druckempfindlichen Widerstand zum Messen oder Sammeln von Informationen über den Druck an jeder Fingerspitze der menschlichen Hand, einen Beschleunigungsmesser zum Messen der Beschleunigungskräfte der menschlichen Hand und Finger und/oder ein Gyroskop zum Messen oder Beibehalten der Ausrichtung und der Winkelgeschwindigkeit der menschlichen Hand und Finger; einen Mikrocontroller, der konfiguriert ist, um die Bewegung der Vielzahl von Manipulationseinheiten (100, 200, 593) in Reaktion auf Daten zu steuern/manipulieren, koordinieren und/oder synchronisieren, die von dem einen oder den mehreren Sensoren gemessen und bereitgestellt sind, um gewünschte Betätigungen der Roboterhand zur Unterstützung oder Verhinderung der Bewegung der menschlichen Hand durchzuführen.

8. - Roboterhand oder Roboterhandschuh nach Anspruch 7, wobei die Handflächenplatte weiter mindestens einen ersten Befestigungsverbinder mit einem ersten Eingriffselement umfasst, das an oder in der Nähe eines proximalen Endes der Handflächenplatte angeordnet ist, um mit einem menschlichen Handgelenk und/oder Unterarm und/oder mindestens einem zweiten Befestigungsverbinder zu koppeln, der ein zweites Eingriffselement aufweist, das an oder in der Nähe eines distalen Endes der Handflächenplatte angeordnet ist, um mit dem ersten Verbinder (161, 261, 361, 561) einer proximalen der Vielzahl von Manipulationseinheiten (100, 200, 593) zu koppeln.

9. - Roboterhand oder Roboterhandschuh nach Anspruch 7, wobei zwei Manipulationseinheiten (100, 200, 593), die auf eine abgestufte Weise angeordnet sind, mit einem der Finger oder Daumen der menschlichen Hand koppeln, um 2 Freiheitsgrade bereitzustellen, um die Drehung eines oder mehrere Fingergelenke zu betätigen, darin eingeschlossen das PIP-Gelenk und/oder das MCP-Gelenk, um eine Flexion und/oder Extension des Fingers oder Daumens bereitzustellen, und/oder zwei oder drei Manipulationseinheiten (100, 200, 593), die auf eine abgestufte Weise angeordnet sind, um mit anderen der Finger koppeln, darin eingeschlossen den Zeigefinger, Mittelfinger, Ringfinger und kleinen Finger der menschlichen Hand, um 2 oder 3 Freiheitsgrade bereitzustellen und die Drehung eines oder mehrerer Fingergelenke zu betätigen, darin eingeschlossen das PIP-Gelenk, MCP-Gelenk und/oder DIP-Gelenk; oder des Daumens der menschlichen Hand, um 2 oder 3 Freiheitsgrade bereitzustellen und die Drehung eines oder mehrerer Fingergelenke zu betätigen, darin eingeschlossen das IP-Gelenk, MCP-Gelenk und/oder CMC-Gelenk; und/oder wobei zusätzlich ein oder mehrere Manipulatoren allein oder in Kombination mit anderen mit dem Daumen oder anderen Fingern koppeln, um einen zusätzlichen Freiheitsgrad zur Abduktion und/oder Adduktion bereitzustellen.

10. - Roboterhand oder Roboterhandschuh nach einem der Ansprüche 7 - 9, wobei jede der Manipulationseinheiten (100, 200, 593) konfiguriert ist, um sich selbst oder einem Segment eines assoziierten Fingers der menschlichen Hand eine Drehbewegung bereitzustellen, die vorzugsweise grösser als 0 Grad und bevorzugter kleiner als 180 Grad ist.

## Revendications

1. - Ensemble d'entraînement pour déplacer ou faire tourner, ou assister ou résister au mouvement d'une première partie de corps par rapport à une seconde partie de corps et, de préférence, permettre la flexion, l'extension, la pronation, la supination, la rotation, l'abduction et l'adduction de la première partie de corps par rapport à la seconde partie de corps, comprenant au moins une unité de manipulation cascadable à centre à distance (100, 200, 593) pour partie de corps, comprenant un boîtier (110, 210, 510) pour recevoir au moins en partie un actionneur (220, 520), un engrenage de transmission de couple/force (230, 530), une roue dentée partielle (240, 540) reliés de manière fonctionnelle les uns aux autres, un premier connecteur (161, 261, 361, 561) agencé pour un accouplement à la première partie de corps, un second connecteur (162, 262, 362, 562) couplé à la roue dentée partielle (240, 540) et agencé pour un accouplement à la seconde partie de corps et configuré en outre pour être entraîné par la roue dentée partielle actionnée par l'actionneur (220, 520) par l'intermédiaire de l'engrenage de transmission de couple (230, 530) pour un accouplement et un déplacement avec la seconde partie de corps, et un canal ou rail en arc (250, 550) pour confiner et/ou guider un mouvement de rotation réciproque ou en va-et-vient, à centre à distance, de la roue dentée partielle (240, 540), le second connecteur (162, 262, 362, 562) étant configuré pour être entraîné par la roue dentée partielle (240, 540) pour tourner d'un premier angle prédéterminé ou souhaité autour d'un axe de rotation défini par une courbure de la roue dentée partielle et du canal ou rail en arc (250, 550) en réponse à une instruction prédéfinie ou à une instruction reçue en temps réel, de façon à entraîner la seconde partie de corps accouplée à celui-ci pour tourner d'un second angle prédéterminé ou souhaité autour d'une articulation du corps positionnée entre les première et seconde parties de corps,
l'unité de manipulation cascadable est capable de faire tourner la seconde partie de corps sans occuper un espace axial de l'articulation du corps ; et
l'unité de manipulation cascadable étant cascadable avec son double ou une autre unité de manipulation cascadable dotée d'actionneurs différents, d'engrenages différents, de tailles différentes, de plages de mouvement différentes ou de matériaux différents, pour la manipulation de multiples articulations du corps ou la manipulation de mouvements composites d'une seule articulation du corps.

2. - Ensemble d'entraînement selon la revendication 1, comprenant une pluralité d'unités de manipulation cascadables (100, 200, 593) de dimensions identiques ou de dimensions différentes, disposées en série et/ou en parallèle les unes avec les autres ; des premiers connecteurs respectifs (161, 261, 361, 561) de la pluralité d'unités de manipulation (100, 200, 593) étant configurés pour s'accoupler entre eux et/ou à la même première partie de corps, et des seconds connecteurs respectifs (162, 262, 362, 562) de la pluralité d'unités de manipulation étant configurés pour s'accoupler entre eux et/ou à la même seconde partie de corps pour fournir des degrés de liberté supplémentaires de rotation ou de mouvement pour la seconde partie de corps par rapport à la première partie de corps ; et/ou
le second connecteur (162, 262, 362, 562) de l'une de la pluralité d'unités de manipulation (100, 200, 593) est configuré pour s'accoupler au premier connecteur (161, 261, 361, 561) d'une autre de la pluralité d'unités de manipulation, de façon à fournir un angle de rotation supplémentaire et/ou des degrés de liberté supplémentaires de mouvement pour une ou plusieurs parties de corps ou articulations accouplées à une ou plusieurs de la pluralité d'unités de manipulation et entraînées individuellement/respectivement ou collectivement par celles-ci.

3. - Ensemble d'entraînement selon la revendication 1 ou 2, comprenant en outre une ou plusieurs butées disposées au niveau, et de préférence de manière adjacente à une extrémité arrière, de la roue dentée partielle, du canal ou rail en arc, et/ou du boîtier pour confiner le mouvement et/ou fournir des limites de sécurité pour le mouvement de la roue dentée partielle.

4. - Ensemble d'entraînement selon la revendication 1 ou 2, dans lequel l'actionneur est choisi parmi un groupe comprenant un moteur CC, un moteur CA/CC, un moteur hydraulique, un moteur pneumatique, un moteur à balais/sans balais, un moteur piézo, un alliage à mémoire de forme actionné par de l'électricité CA/CC, des piles, des produits chimiques, des changements de température, des changements de pression, des lumières ou de l'électromagnétisme, du son ou des ondes énergétiques ; et/ou
dans lequel l'engrenage de transmission de couple est choisi parmi un groupe comprenant une vis sans fin, une roue droite cylindrique, un engrenage conique, un engrenage à courroie, un engrenage à poulie et une combinaison de ceux-ci ; et/ou
dans lequel la roue dentée partielle est choisie parmi un groupe comprenant une vis sans fin, une roue droite cylindrique, une roue conique, une roue à courroie, une roue à poulie et une combinaison de celles-ci ; et/ou dans lequel la roue dentée partielle est une roue dont la rotation est de moins de 360 degrés, de préférence de moins 135 degrés, mais de plus de 0 degré.

5. - Ensemble d'entraînement selon la revendication 1, dans lequel l'unité de manipulation (100, 200, 593) comprend en outre un ou plusieurs capteurs et/ou un ou plusieurs contrôleurs disposés sur ou dans le boîtier pour mesurer ou contrôler / traiter des données relatives à des positions, angles de rotation, degrés de mouvement ou orientations du second connecteur (162, 262, 362, 562), à des forces ou pressions exercées ou reçues par le second connecteur (162, 262, 362, 562), à un son ambiant, à la lumière et/ou à la température ;
des données mesurées et de sortie ou de retour générées par les capteurs et/ou les contrôleurs sont communiquées ou transmises, de préférence à un terminal distant/externe, via des technologies ou protocoles filaires et/ou sans fil, notamment Bluetooth, Wifi, infrarouge, Zigbee, GSM, LTE et une combinaison de ceux-ci.

6. - Ensemble d'entraînement selon la revendication 1, dans lequel une paire adjacente d'une première et d'une seconde unité de manipulation cascadable (100, 200, 593) sont accouplées l'une à l'autre via une interconnexion du second connecteur (162, 262, 362, 562) de la première unité de manipulation et du premier connecteur (161, 261, 361, 561) de la seconde unité de manipulation, une interconnexion du premier connecteur (161, 261, 361, 561) de la première unité de manipulation et du second connecteur (162, 262, 362, 562) de la seconde unité de manipulation, une interconnexion du premier connecteur (161, 261, 361, 561) de la première unité de manipulation et du premier connecteur (161, 261, 361, 561) de la seconde unité de manipulation, ou une interconnexion du second connecteur (162, 262, 362, 562) de la première unité de manipulation et du second connecteur (162, 262, 362, 562) de la seconde unité de manipulation.

7. - Main ou gant robotique (500) pour déplacer ou faire tourner, ou assister ou résister au mouvement d'une main humaine et/ou de ses doigts, comprenant un ensemble d'entraînement selon l'une quelconque des revendications 1 à 6, comprenant en outre une plaque de paume pour monter la main ou gant robotique sur le poignet humain, et/ou un avant-bras et pour monter au moins une unité de manipulation cascadable à centre à distance (100, 200, 593) pour l'utilisation et la manipulation d'au moins un doigt de la main humaine, chacun de la pluralité de doigts étant accouplé à et actionné par une ou plusieurs unités de manipulation (100, 200, 593) configurées pour et capables de fournir un ou plusieurs degrés de liberté ; et
un ou plusieurs capteurs ou dispositifs de détection comprenant un capteur électromyographique pour mesurer une activité électrique de muscles de la main humaine et des doigts, une résistance sensible à la force pour mesurer ou recueillir des informations concernant la pression à chaque extrémité de doigt de la main humaine, un accéléromètre pour mesurer des forces d'accélération de la main humaine et des doigts, et/ou un gyroscope pour mesurer ou maintenir une orientation et une vitesse angulaire de la main humaine et des doigts ;
un microcontrôleur configuré pour contrôler / manipuler, coordonner et/ou synchroniser le mouvement de la pluralité d'unités de manipulation (100, 200, 593) en réponse à des données mesurées et fournies par le ou les capteurs pour effectuer des opérations souhaitées de la main robotique pour assister ou résister au mouvement de la main humaine.

8. - Main ou gant robotique selon la revendication 7, dans lequel la plaque de paume comprend en outre au moins un premier connecteur de fixation ayant un premier élément d'engagement disposé à ou près d'une extrémité proximale de la plaque de paume pour un accouplement à un poignet et/ou un avant-bras humain et/ou au moins un second connecteur de fixation ayant un second élément d'engagement disposé à ou près d'une extrémité distale de la plaque de paume pour un accouplement au premier connecteur (161, 261, 361, 561) d'une unité de manipulation proximale parmi la pluralité d'unités de manipulation (100, 200, 593).

9. - Main ou gant robotique selon la revendication 7, dans lequel deux unités de manipulation (100, 200, 593) disposées en cascade sont accouplées à l'un des doigts ou au pouce de la main humaine pour fournir 2 degrés de liberté et actionner la rotation d'une ou plusieurs articulations de doigt, y compris l'articulation PIP et/ou l'articulation MCP pour fournir une flexion et/ou extension du doigt ou du pouce ; et/ou deux ou trois unités de manipulation (100, 200, 593) disposées en cascade sont accouplées à un autre des doigts, y compris l'index, le majeur, l'annulaire et l'auriculaire, de la main humaine pour offrir 2 ou 3 degrés de liberté et actionner la rotation d'une ou plusieurs articulations de doigt, y compris l'articulation PIP, l'articulation MCP et/ou l'articulation DIP ; ou le pouce de la main humaine pour fournir 2 ou 3 degrés de liberté et actionner la rotation d'une ou plusieurs articulations de doigt, y compris l'articulation IP, l'articulation MCP et/ou l'articulation CMC ; et/ou un ou plusieurs manipulateurs supplémentaires disposés de manière autonome ou en combinaison avec d'autres sont accouplés au pouce et à d'autres doigts pour fournir un degré de liberté supplémentaire pour l'abduction et/ou l'adduction.

10. - Main ou gant robotique selon l'une quelconque des revendications 7 à 9, dans lequel chacune des unités de manipulation (100, 200, 593) est configurée pour se donner à elle-même ou à un segment d'un doigt associé de la main humaine un mouvement de rotation de préférence de plus de 0 degré et de façon davantage préférée de moins de 180 degrés.
